# EUROPEAN PATENT SPECIFICATION

(11) **EP 1 334 698 B1**
(45) Date of publication and mention of the grant of the patent: **08.11.2006**
(21) Application number: 03002589.4
(22) Date of filing: 07.02.2003
(51) Int. Cl.: A61B 17/70

(54) **Rod distance retainer**
Distanzhalter für Stäbe
Element d'écartement pour tiges

(30) Priority: 08.02.2002 JP 2002033040
(43) Date of publication of application: 13.08.2003
(73) Proprietor: Showa IKA Kohgyo Co., Ltd., Aichi-ken (JP)
(72) Inventor: Oribe, Kazuya, Minato-ku, Tokyo (JP); Takamido, Hiroshi, Nagoya-shi Aichi-ken (JP)
(74) Representative: Zimmer, Franz-Josef

(56) References cited:
- EP-A- 0 953 316
- WO-A-00/59387
- WO-A-95/13754

## Description

The present invention relates to a rod distance retainer according to the preamble part of the independent claim 1. More specifically, the present invention relates to a rod distance retainer for retaining a pair of rods which are fixed appropriately onto a plurality of vertebral bodies so as to constantly retain a distance between the plurality of vertebral bodies being spaced appropriately.

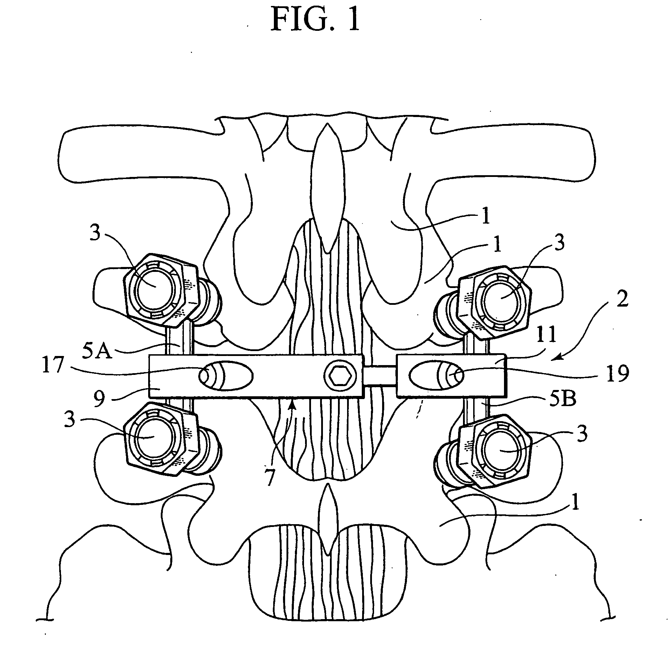

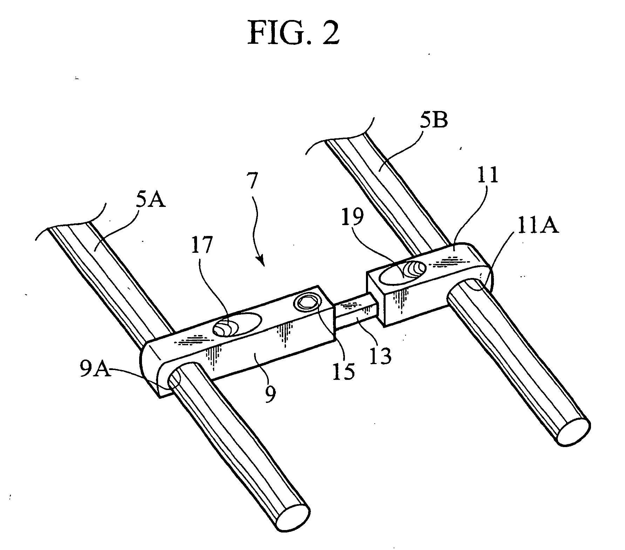

A vertebral body retainer 2 shown in FIGs. 1 and 2 is known as the related art for retaining vertebral bodies which are appropriately spaced. The vertebral body retainer 2 includes a pair of rods 5A and 5B, a plurality of implants 3, and a coupler link 7. Both end portions of the pair of rods 5A and 5B are fixed to a plurality of vertebral bodies 1, which are appropriately spaced, by use of the plurality of implants 3 such as screws. Thereby, a distance between the pair of vertebral bodies 1 is retained constantly. Moreover, a constitution of coupling the rods 5A and 5B with the coupler link 7 is adopted so as to constantly retain the space between the pair of rods 5A and 5B.

FIG. 2 shows a structure of the coupling link 7 of the vertebral body retainer 2. Specifically, the coupler link 7 includes a fixed link 9 provided with a semicircular engaging concave portion 9A on a tip portion thereof so as to be engageable with the rod 5A, and a movable link 11 provided with a semicircular engaging concave portion 11A on a tip portion thereof so as to be engageable with the rod 5B. Moreover, the fixed link 9 and the movable link 11 are retractably coupled together.

Specifically, an inserting rod 13 with a quadrangular cross section formed on a base end side of the movable link 11 is movably inserted into an engaging hole (not shown) with a quadrangular cross section formed as an opening on a base end side of the fixed link 9. Then, the inserting rod 13 is fixed by a fixing screw 15 being screwed into the fixed link 9. Therefore, it is possible to coordinate the engaging concave portions 9A and 11A of the both links 9 and 11 with the distance between the both rods 5A and 5B in the state of loosening the fixing screw 15.

To fix the rods 5A and 5B to the engaging concave portions 9A and 11A, tapped holes 17 and 19 for allowing insertion of fixing screws (not shown) are formed tapped holes 17 and 19 for allowing insertion of fixing screws (not shown) are formed on the both links 9 and 11, respectively so as to communicate with the engaging concave portions 9A and 11A.

However, in the coupler link 7 proposed in the foregoing description, diameters of the engaging concave portions 9A and 11A are formed substantially equal to diameters of the rods 5A and 5B, and the movable link 11 is not rotatable with respect to the fixed link 9. Accordingly, if any one of the rods 5A and 5B is slightly inclined, there is a problem that the coupler link 7 is difficult to deal with such inclination.

Moreover, since the tapped holes 17 and 19 are formed obliquely against upper surfaces of the fixed link 9 and the movable link 11, formation of the tapped holes 17 and 19 becomes complicated and the fixing screws need to be rotated in oblique directions. Thus, the coupler link 7 has a problem in terms of operability. Furthermore, the fixing screws to be screwed into the tapped holes 17 and 19 have structures to press the rods 5A and 5B by flat faces on tips thereof from orthogonal directions with respect to shaft centers of the rods 5A and 5B, therefore the fixing screws constitute line contacts. In this regard, an improvement for tighter fixation is requested.

WO 00 59387 A discloses a transconnector for coupling spinal rods including a male member, a female member, and a locking member. The male member and the female member are secured to each other by the locking member. Here, both the male member and the female member comprise at their one end being opposite to the connecting portion between the two members a linking element with a space for receiving a fixation element. The fixation elements are secured in the linking elements, respectively, by clamping screws, wherein in the secured state the fixation elements fill in accurate to size of the space of the linking elements, respectively.

From WO 95 13754 A a transverse link for a spinal implant system is shown, wherein the transverse link comprises comparable elements to those of the transconnector of WO 00 59387 A.

It is an objective of the present invention to improve a rod distance retainer as indicated above so as to be excellent in operability and workability.

The objective is solved according to the present invention by a rod distance retainer, comprising a coupler link including rod engaging concave portions which engage a pair of rods, wherein the rods are adapted to fix a plurality of adjacent vertebral bodies, and fixing screws which fix the rods to the rod engaging concave portions, wherein taper faces are formed on tip portions of the fixing screws to enable the fixing screws to abut on peripheral surfaces of the rods, and wherein each of the rod engaging concave portions is formed as a long hole elongated along a moving direction of the respective fixing screw.

Further preferred embodiments of the present invention are laid down in the further subclaims.

In the following, the present invention is explained in greater detail by means of several embodiments thereof in conjunction with the accompanying drawings, wherein:
FIG. 1 is a schematic view showing a structure of a proposed vertebral body retainer;
FIG. 2 is a perspective view showing the structure of the proposed rod distance retainer shown in FIG. 1;
FIG. 3 is a schematic view showing a structure of a vertebral body retainer according to an embodiment of the present invention;
FIG. 4A is a top plan view showing a structure of a rod distance retainer according to an embodiment of the present invention; and
FIG. 4B is a cross-sectional view showing the structure of the rod distance retainer shown in FIG. 4A.

Hereinafter, description will be made of embodiments of the present invention with reference to the drawings.

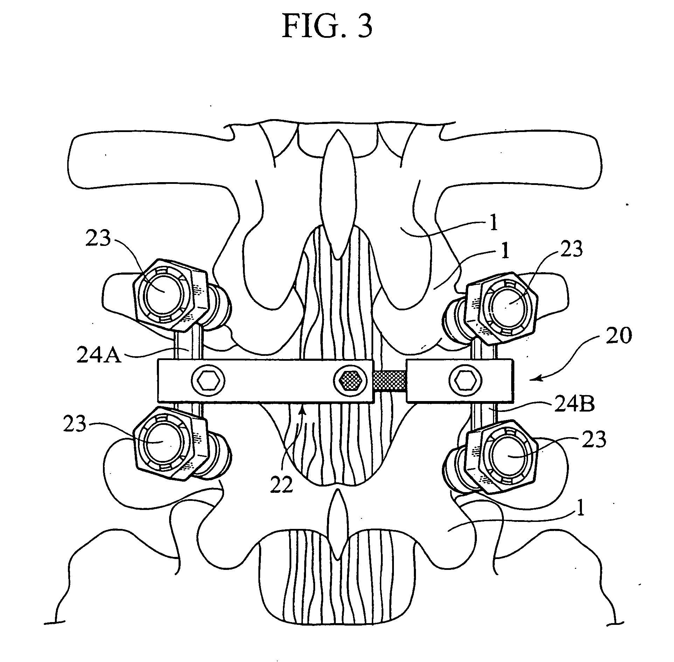

FIG. 3 shows a vertebral body retainer 20 using a rod distance retainer according to an embodiment of the present invention. The vertebral body retainer 20 mainly includes a pair of rods 24A and 24B, a plurality of implants 23, and a coupler link 22. Both end portions of the pair of rods 24A and 24B are fixed to a plurality of vertebral bodies 1, which are appropriately spaced, by use of the plurality of implants 23 such as screws, and a distance between the pair of vertebral bodies 1 is thereby retained constantly. Moreover, a constitution of coupling the rods 24A and 24B with the coupler link 22 is adopted so as to constantly retain the space between the pair of rods 24A and 24B.

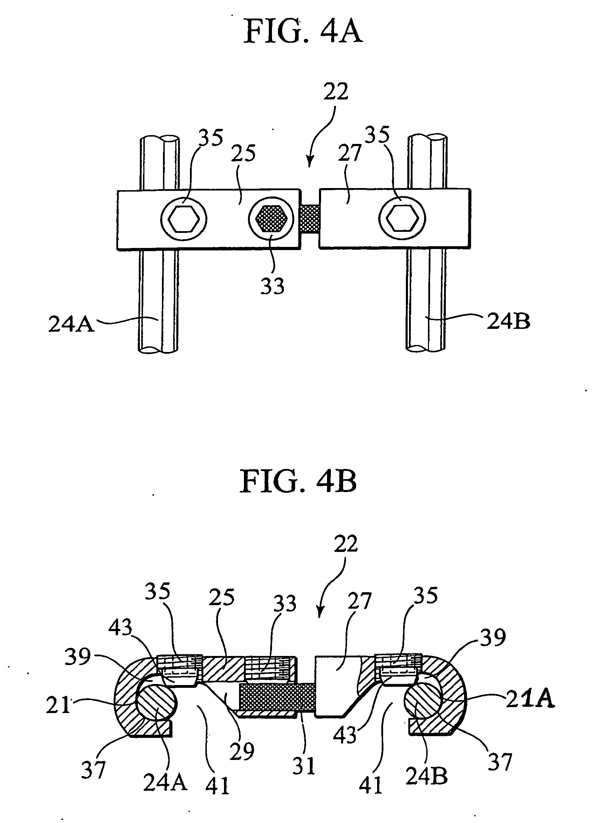

FIGs. 4A and 4B shows a constitution of the rod distance retainer, which comprises the coupler link 22. The coupler link 22 is constituted by rod engaging concave portions 21 and 21A being provided on both ends of the coupler link 22, a fixed link 25 provided with the rod engaging concave portion 21 on a tip portion thereof, and a movable link 27 provided with the rod engaging concave portion 21A on a tip portion thereof. A columnar inserting rod 31 provided on a base end portion of the movable link 27 is movably and rotatably fitted into an engaging hole 29 which is formed along the longitudinal direction on the fixed link 25. Moreover, the inserting rod 31 is fixed by a fixing screw 33 which is screwed into the fixed link 25.

Therefore, when fixation of the inserting rod 31 by the fixing screw 33 is released, the inserting rod 31 can be moved in the axial direction along the engaging hole 29. Accordingly, it is possible to coordinate a distance between the rod engaging concave portions 21 and 21A with a distance between the pair of rods 24A and 24B and also to rotate the movable link 27 with respect to the fixed link 25. Hence, if the rod 24B is slightly inclined against the rod 24A, it is possible to engage the rod engaging concave portions 21 and 21A to the both rods 24A and 24B respectively in an aligned state.

The engaging concave portions 21 and 21A are formed symmetrically. Each of the engaging concave portions 21 and 21A includes an arc-shaped support 37 for supporting the rod 24A or 24B when a fixing screw 35 for rod-fixing is screwed into the fixed link 25 or the movable link 27 so as to fix the rod 24A or 24B by pressure. Moreover, each of the engaging concave portions 21 and 21A includes a clearance 39 on an upper part of the support 37 for allowing upward movement of the rod 24A or 24B in the event of loosening the fixing screw 35. Furthermore, apertures 41 are formed obliquely toward inner lower sides, so as to communicate with the rod engaging concave portions 21 and 21A, respectively.

In other words, each of the rod engaging concave portions 21 and 21A is formed as a long hole along the moving direction of the fixing screw 35, i.e. in the vertical direction in FIG. 4B, and is arranged to communicate with the aperture 41 on one side of this long hole.

When the rods 24A and 24B are engaged with the rod engaging concave portions 21 and 21A, each of the fixing screw 35 is located in a position which is shifted inward from the shaft center of the rod 24A or 24B, i.e. toward the aperture 41 side. Moreover, the fixing screws 35 are screwed into tapped holes, which are formed in the orthogonal direction to upper surfaces of the fixed link 25 and the movable link 27 respectively, at communicating portions of the rod engaging concave portions 21 and 21 A with the apertures 41. Furthermore, a tip portion of the fixing screw 35 is formed into a tapered shape having a taper face 43, so as to be capable of abutting on the outer periphery of the rod 24A or 24B which is located in the rod engaging concave portion 21 or 21A.

In the vertebral body retainer 20 of the above-described constitution, as shown in FIGs. 4A and 4B, the rod engaging concave portions 21 and 21A provided on the both ends of the coupler link 22 are engaged with the pair of rods 24A and 24B, which are integrated with the vertebral bodies 1 by use of the plurality of implants 23. Here, even if the rod 24B is slightly inclined against the rod 24A, it is possible to engage the both rods easily in response to such inclination because the clearances 39 are provided on the rod engaging concave portions 21 and 21A, and because the movable link 27 is arranged as rotatable relatively to the fixed link 25.

The distance between the rod engaging concave portions 21 and 21A is adjusted in response to the distance between the pair of rods 24A and 24B. Thereafter, the inserting rod 31 is fixed by the fixing screw 33 so as to integrate the fixed link 25 with the movable link 27, and then the fixing screws 35 are tighten up. In this way, the rods 24A and 24B can be surely fixed into the rod engaging concave portions 21 and 21A.

When the fixing screws 35 are tightened up, the taper faces 43 provided on the tip portions of the fixing screws 35 abut on the outer peripheries of the rods 24A and 24B respectively, and act to press the rods 24A and 24B obliquely downward. Accordingly, the rods 24A and 24B can be surely fixed by pressure into inner peripheries of the rod engaging concave portions 21 and 21A.

In this event, since the taper face 43 of the fixing screw 35 contacts with the outer periphery of the rod 24A or 24B, large pressure is generated by a wedge effect, and the contact position constitutes a point contact. Accordingly, the contact portion will receive substantial stress. Hence, the contact portion is elastically deformed and is prevented from loosening. Meanwhile, reactive force acts on the tip portion of the fixing screw 35 upon tightening, so as to slant the fixing screw 35 against the tapped hole. Accordingly, the fixing screws 35 are prevented from loosening inside the tapped holes, whereby the rods 5Aand 5B can be surely and tightly fixed.

According to the rod distance retainer of this embodiment, the taper face provided on the tip portion of the fixing screw is allowed to abut on the outer periphery of the rod so as to fix the rod by pressure to the rod engaging concave portion. Therefore, the rod can be surely and tightly fixed by pressure. As a consequence, the vertebral bodies which are engaged with the rod can be surely fixed. Moreover, the workability is improved because the tapped holes on the coupler link do not have to be formed obliquely. In addition, the operability is also improved because the fixing screws do not have to be rotated in the oblique directions.

Although the invention has been described above by reference to certain embodiments of the invention, the invention is not limited to the embodiments described above will occur to these skilled in the art, in light of the teachings. The scope of the invention is defined with reference to the following claims.

## Claims

1. A rod distance retainer, comprising:
a coupler link (22) including rod engaging concave portions (21, 21A) which engage a pair of rods (24A, 24B), wherein the rods (24A, 24B) are adapted to fix a plurality of adjacent vertebral bodies (1); and
fixing screws (35) which fix the rods (24A,24B) to the rod engaging concave portions (21, 21A), wherein taper faces (43) are formed on tip portions of the fixing screws (35) to enable the fixing screws (35) to abut each via the taper face on peripheral surfaces of the rods (24A, 24B);
**characterized in that** each of the rod engaging concave portions (21, 21A) is formed as a long hole elongated along a moving direction of the respective fixing screw (35).

2. A rod distance retainer according to claim 1, wherein the fixing screws (35) are located respectively in a position being shifted from a shaft center of the respective rods (24A, 24B) toward an aperture (41) of the respective rod engaging concave portions (21, 21A).

3. A rod distance retainer according to claim 1 or 2, wherein clearances (39) are provided on the rod engaging concave portions (21, 21A) respectively, wherein the clearances (39) are adapted to allow movement of the rods (24A, 24B) in an inserting/pulling direction of the fixing screw (35).

4. A rod distance retainer according to one of the claims 1 to 3, wherein the coupler link (22) includes a fixed link (25) and a movable link (27), wherein the movable link (27) is detachably and rotatably fitted into the fixed link (25).

5. A rod distance retainer according to one of the claims 1 to 4, wherein the fixing screws (35, 33) are screwed in an orthogonal direction with respect to an upper surface of the coupler link (22).

## Patentansprüche

1. Stangenabstandshalter, aufweisend:
eine Kupplungsverbindung (22), die konkave Stangen- Eingriffsabschnitte (21, 21A) enthält, die mit einem Stangenpaar (24A, 24B) im Eingriff sind, wobei die Stangen (24A, 24B) vorgesehen sind, eine Mehrzahl von benachbarten Wirbelkörpern (1) zu fixieren; und
Befestigungsschrauben (35), die die Stangen (24A, 24B) an den konkaven Eingriffsabschnitten (21, 21A) befestigen, wobei keilförmige Flächen (43) an Spitzenabschnitten der Befestigungsschrauben (35) ausgebildet sind, um die Befestigungsschrauben (35) in die Lage zu versetzen, jeweils über die keilförmige Fläche an den Umfangsoberflächen der Stangen (24A, 24B) anzuliegen;
**dadurch gekennzeichnet, dass** jeder der konkave Stangen- Eingriffsabschnitte (21, 21A) als ein Langloch, lang gestreckt entlang einer Bewegungsrichtung der jeweiligen Befestigungsschraube (35), ausgebildet ist.

2. Stangenabstandshalter nach Anspruch 1, wobei die Befestigungsschrauben (35) jeweils in einer Position platziert sind, die gegenüber einer Wellenmitte der jeweiligen Stangen (24A, 24B) in die Richtung zu einer Öffnung (41) der jeweiligen konkaven Stangen- Eingriffsabschnitte (21, 21A) versetzt ist.

3. Stangenabstandshalter nach Anspruch 1 oder 2, wobei jeweils Spalte (39) an den konkaven Stangen- Eingriffsabschnitten (21, 21A) vorgesehen sind, wobei die Spalte (39) vorgesehen sind, die Bewegung der Stangen (24A, 24B) in einer Einsetz- / Herausziehrichtung der Befestigungsschraube (35) zu gestatten.

4. Stangenabstandshalter nach einem der Ansprüche 1 bis 3, wobei die Kupplungsverbindung (22) eine feste Verbindung (25) und eine bewegbare Verbindung (27) enthält, wobei die bewegbare Verbindung (27) lösbar ist und in der feststehenden Verbindung (25) drehbar eingesetzt ist.

5. Stangenabstandshalter nach einem der Ansprüche 1 bis 4, wobei die Befestigungsschrauben (35, 33) in einer rechtwinkligen Richtung in Bezug auf eine obere Oberfläche der Kupplungsverbindung (22) verschraubt sind.

## Revendications

1. Elément de maintien de distance pour tiges, comprenant :
une pièce intermédiaire de couplage (22) comprenant des parties concaves d'engagement de tige (21, 21A) qui engagent une paire de tiges (24A, 24B), dans laquelle les tiges (24A, 24B) sont adaptées pour fixer une pluralité de corps vertébraux adjacents (1) ; et
des vis de fixation (35) qui fixent les tiges (24A, 24B) aux parties concaves d'engagement de tige (21, 21A), dans lequel des faces coniques (43) sont formées sur des parties d'extrémité des vis de fixation (35) afin de permettre aux vis de fixation (35) de venir en butée chacune par la face conique sur des surfaces périphériques des tiges (24A, 24B) ;
**caractérisé en ce que** chacune des parties concaves d'engagement de tige (21, 21A) est formée comme un trou long s'allongeant le long d'une direction de déplacement de la vis de fixation respective (35).

2. Elément de maintien de distance pour tiges selon la revendication 1, dans lequel les vis de fixation (35) sont situées respectivement dans une position étant décalée d'un centre d'arbre des tiges respectives (24A, 24B) vers une ouverture (41) des parties concaves d'engagement de tige respectives (21, 21A).

3. Elément de maintien de distance pour tiges selon la revendication 1 ou 2, dans lequel des espaces (39) sont prévus sur les parties concaves d'engagement de tige (21, 21A) respectivement, dans lequel les espaces (39) sont adaptés pour permettre un mouvement des tiges (24A, 24B) dans une direction d'insertion / de traction de la vis de fixation (35).

4. Elément de maintien de distance pour tiges selon l'une des revendications 1 à 3, dans lequel la pièce intermédiaire de couplage (22) comprend une pièce intermédiaire fixe (25) et une pièce intermédiaire mobile (27), dans lequel la pièce intermédiaire mobile (27) est ajustée de manière tournante et de manière amovible dans la pièce intermédiaire fixe (25).

5. Elément de maintien de distance pour tiges selon l'une des revendications 1 à 4, dans lequel les vis de fixation (35, 33) sont vissées dans une direction orthogonale par rapport à une surface supérieure de la pièce intermédiaire de couplage (22).
